# FASCICULE DE BREVET EUROPEEN

(11) **EP 1 639 964 B1**
(45) Date de publication et mention de la délivrance du brevet: **04.06.2008**
(21) Numéro de dépôt: 05017693.2
(22) Date de dépôt: 13.08.2005
(51) Int. Cl.: A61F 2/30

(54) **Tige fémorale pour prothèse de hanche**
Femurschaftprothese für die Hüfte
Femoral stem for a hip prosthesis

(30) Priorité: 27.09.2004 FR 0410392
(43) Date de publication de la demande: 29.03.2006
(73) Titulaire: CENTERPULSE FRANCE SAS, 25461 Etupes Cedex (FR); HIP CARE, 58000 Nevers (FR)
(72) Inventeur: Bizot, Pascal, 25000 Besancon (FR); Boisgard, Stéphane, 25000 Besancon (FR); De Tiesenhausen, Nicolas, 25000 Besancon (FR); Garbuio, Patrick, 25000 Besancon (FR); Gouin, François, 25000 Besancon (FR); Morin, Olivier, 25000 Besancon (FR); Nizard, Rémy, 25000 Besancon (FR); Ourcival, Serge, 25000 Besancon (FR); Ovadia, Hervé, 25000 Besancon (FR); Paris, David, 25000 Besancon (FR); Polard, Jean-Louis, 25000 Besancon (FR); Sollogoub, Yvan, 25000 Besancon (FR); Tarle, Olivier, 25000 Besancon (FR); Viale, Patrick, 25000 Besancon (FR); Bouraly, Jean-Pierre, 25000 Besancon (FR)
(74) Mandataire: Bentz, Jean-Paul

(56) Documents cités:
- FR-A- 2 784 576
- FR-A- 2 827 155
- FR-A- 2 851 456

## Description

La présente invention concerne une tige fémorale pour prothèse de hanche destinée à être implanter avec ou sans ciment dans un fût fémoral.

Une telle tige est constituée par une partie distale diaphysaire de forme allongée, destinée à être engagée dans la cavité médullaire d'un fémur, avec ou sans ciment, et d'une partie métaphysaire dont l'extrémité libre, ou col est munie d'une tête apte à s'articuler dans une cupule de la cavité cotyloïdienne.

Dans le cas d'une tige sans ciment, la fixation est réalisée en deux étapes, à savoir une fixation primaire par blocage mécanique de l'implant directement en contact partiel avec la surface interne du canal, et une fixation secondaire par repousse osseuse au niveau de l'interface.

L'utilisation d'un matériau intrinsèquement réhabitable, tel qu'un alliage de titane par exemple, permet à la repousse osseuse de venir épouser de manière intime le relief de la tige et ainsi de renforcer l'immobilisation de l'implant même si son état de surface est lisse. La combinaison des fixations primaire et secondaire garantit la pérennité du blocage.

Dans le cadre des tiges fémorales sans ciment, il est connu de réaliser des micro reliefs sur au moins une des faces de la tige afin de favoriser le blocage primaire et augmenter la surface spécifique de contact afin de favoriser la repousse osseuse, dite « fixation secondaire ».

C'est ainsi qu'il existe de nombreux types de reliefs constitués de stries, notamment en marches d'escalier assurant l'ancrage primaire et empêchant la subsidence de la tige, c'est-à-dire sa descente dans le fût fémoral, à court, moyen et long terme et la repousse osseuse.

Certains types de tiges comportent sur leurs faces antéro postérieures, deux zones de reliefs dont l'une proximale avec des stries transversales en marches d'escalier dans le but d'éviter la subsidence, et une autre distale constituée de canelures longitudinales dont le but est de faciliter le guidage de la tige dans le fût fémoral.

Ce type de tiges est décrit dans les demandes de brevet français N° 2 827 155 et 2 784 576. Mais on peut constater que les stries se répartissent de part et d'autre de la ligne courbe médiane de la tige, ce qui ne permet pas de matérialiser la fibre neutre, pouvant constituer un repère pour le chirurgien lors de l'impactage.

De plus, il a pu être constaté qu'une répartition des stries de part et d'autre de la fibre neutre, plutôt que de la courbe médiane, permettait d'augmenter la résistance à la traction de la tige fémorale.

D'autre part, il est à noter que ces reliefs ou canelures sont réalisés uniquement sur les faces antéro postérieure voire sur la face interne. Force est de constater qu'à ce jour aucune tige prothétique ne comporte de reliefs sur la face externe de celle-ci en contact avec le grand trochanter. Il n'est pas connu non plus de réaliser des reliefs sur les faces antéro postérieure de la tige adaptés aux contraintes de traction liées au grand trochanter. En fait, tous les reliefs connus à ce jour sont réalisés sur les faces antéro postérieure ou sur la face intérieure pour agir et résister à des forces de compression.

En effet, la tige fémorale se présente sous la forme globale d'une potence, à l'extrémité de laquelle est exercée une force provoquant des contraintes au niveau de la face intérieure, alors qu'à l'inverse il se produit un phénomène de tension sur sa face extérieure dans un sens de traction provoquant à moyen et long terme, plus particulièrement pour les tiges sans ciment, une non repousse osseuse au niveau du grand trochanter.

On observe ce phénomène sur les radiographies se traduisant par un liseré qui s'avère évolutif dans le temps et qui est témoin d'une non repousse osseuse faisant craindre un descellement. Ceci est dû au fait que le relief dans cette zone externe n'est pas adapté pour résister aux interférences dues aux micro mouvements complexes du grand trochanter, c'est-à-dire dans un sens de traction et de torsion.

La présente invention a pour but de remédier aux différents inconvénients précités et concerne à cet effet une tige fémorale pour prothèse totale de hanche, constituée par une partie distale diaphysaire de forme allongée destinée à être engagée dans la cavité médullaire d'un fémur, avec ou sans ciment, et d'une partie métaphysaire dont l'extrémité libre est munie d'une tête apte à s'articuler dans une cupule prothétique, des micro-reliefs étant réalisés sur au moins l'une des faces de la tige afin de favoriser la repousse osseuse, caractérisée en ce que ses faces antéro postérieure comportent sur leur partie métaphysaire du côté interne de la fibre neutre, correspondant à l'axe longitudinal de la tige, subissant des efforts de compression, des stries formant un angle inférieur à 90° par rapport à la fibre neutre et se prolongeant du côté externe de la fibre neutre subissant des efforts de traction, par des stries formant un angle sensiblement égal à 90° par rapport à la même fibre neutre.

L'objectif ainsi atteint est celui de résister, à la manière de crochets, à la traction, ce qui aura pour effet de solliciter l'os et de limiter le liseré évoqué ci-dessus.

Selon une autre caractéristique de l'invention, la tige comporte sur sa face externe rejoignant ses faces antéro postérieure, des canelures rectilignes s'étendant sur toute la longueur de la tige. I1 est ainsi obtenu une surface spécifique de contact augmentée, susceptible de favoriser et d'augmenter la repousse osseuse sans qu'il y ait d'interférences dues aux micro mouvements du grand trochanter.

Un autre avantage procuré par l'invention réside dans le fait que les stries situées du côté interne de la fibre étant réalisées selon un certain angle, alors que les stries réalisées du côté externe de la même fibre neutre le sont selon un autre angle, il se produit un effet optique matérialisant cette fibre neutre, ce qui est un repère précis de l'axe de la tige qui, lors de l'impactage, permettra au chirurgien une pose sans valgus et sans varus.

La présente invention concerne également les caractéristiques qui ressortiront au cours de la description qui va suivre, et qui devront être considérées isolément ou selon toutes leurs combinaisons techniques possibles.

Cette description, donnée à titre d'exemple non limitatif, fera mieux comprendre comment l'invention peut être réalisée, en référence aux dessins annexés sur lesquels :

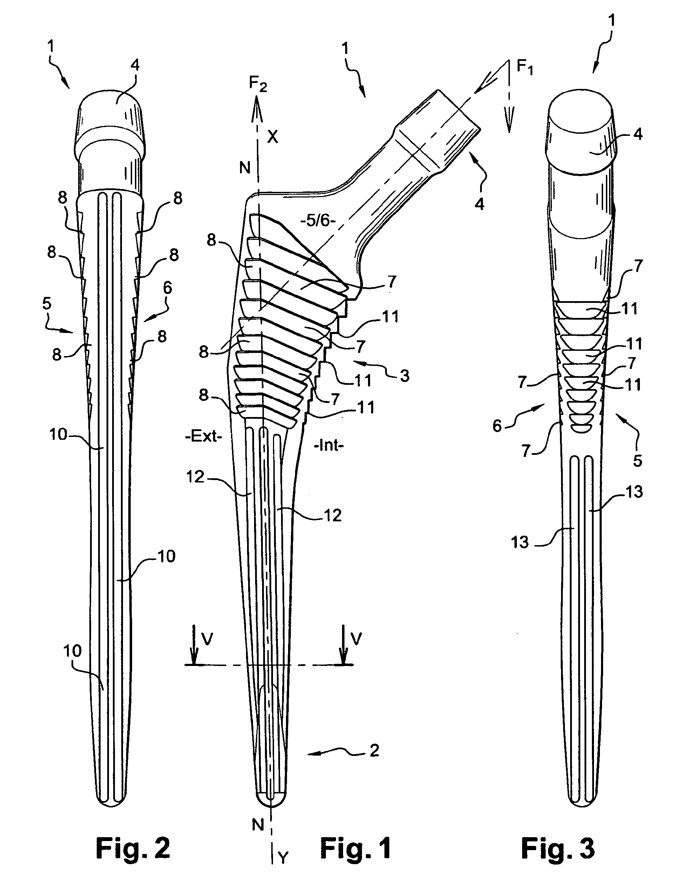

- La figure 1 représente une vue postérieure ou antérieure d'une tige fémorale selon l'invention.
- La figure 2 représente une vue de la face externe de la tige selon la figure 1.
- La figure 3 représente une vue de la face interne de la tige selon la figure 1.

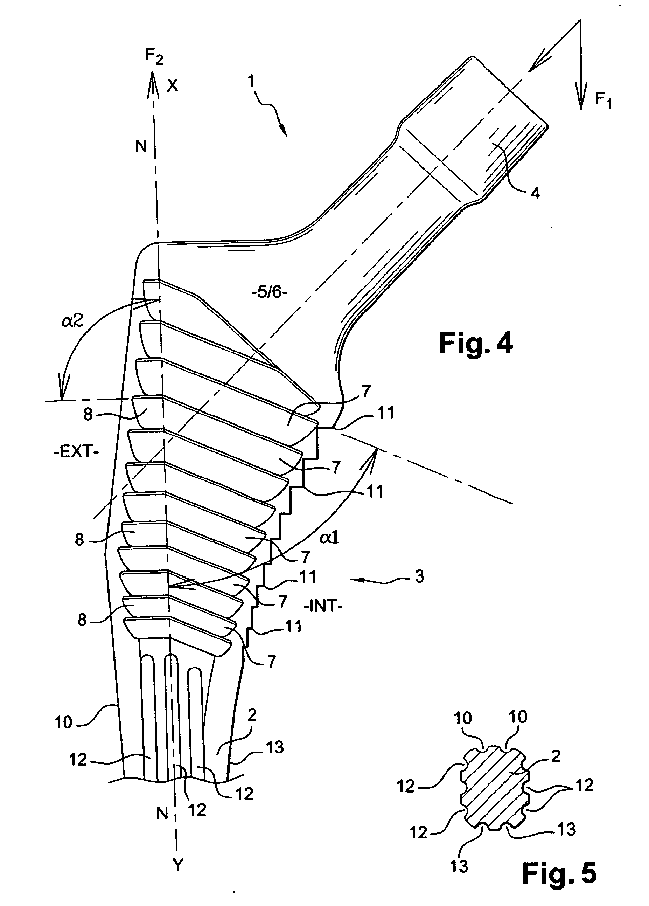

- La figure 4 est une vue à échelle agrandie de la partie métaphysaire de la tige selon la figure 1.
- La figure 5 est une vue en coupe selon la ligne VV de la figure 1.

La tige fémorale 1 représentée sur les figures est constituée par une partie distale diaphysaire, de forme allongée, destinée à être engagée dans la cavité médullaire d'un fémur, avec ou sans ciment, et d'une partie métaphysaire 3 dont l'extrémité libre 4 est munie d'une tête (non représentée) apte à s'articuler dans une cupule de la cavité cotyloïdienne non représentée.

Selon l'invention, la tige 1 comporte sur ses faces antéro postérieure 5, 6 sur sa partie métaphysaire 3 du côté interne INT de la fibre neutre N, correspondant à l'axe longitudinal XY de la tige 1, subissant des efforts de compression F1, des stries 7 formant un angle α1 inférieur à 90° par rapport à la fibre neutre N et se prolongeant du côté externe EXT de la fibre neutre N subissant des efforts de traction F2, par des stries 8 formant un angle α2 sensiblement égal à 90° par rapport à la même fibre neutre N.

Selon une autre caractéristique de l'invention, la tige 1 comporte sur sa face externe EXT rejoignant ses faces antéro postérieure 5, 6, des canelures rectilignes 10 s'étendant sur toute la longueur de la tige 1.

Bien entendu, les deux caractéristiques qui viennent d'être énoncées peuvent être mises en application séparément ou en combinaison.

La tige 1 comporte sur sa face interne INT, dans sa partie métaphysaire 3, des stries transversales 11 sensiblement parallèles entre elles.

Egalement des canelures rectilignes 12 s'étendent sur les faces antéro postérieure 5, 6 dans la zone diaphysaire 2 au-delà des stries transversales 7, 8 de la zone métaphysaire 3.

D'autres canelures rectilignes 13 s'étendent sur la face intérieure INT dans la zone diaphysaire 2 au delà des stries 11 réalisées sur la face intérieure INT.

Il est à préciser enfin que l'invention s'applique autant à une tige droite qu'à une tige anatomique.

## Revendications

1. Tige fémorale (1) pour prothèse totale de hanche, constituée par une partie distale diaphysaire (2) de forme allongée destinée à être engagée dans la cavité médullaire d'un fémur, avec ou sans ciment, et d'une partie métaphysaire (3) dont l'extrémité libre (4) est munie d'une tête apte à s'articuler dans une cupule, des micro-reliefs étant réalisés sur au moins l'une des faces de la tige (1) afin de favoriser la repousse osseuse, **caractérisée en ce que** ses faces antéro postérieure (5, 6) comportent sur leur partie métaphysaire (3) du côté interne (INT) de la fibre neutre (N), correspondant à l'axe longitudinal (XY) de la tige (1), subissant des efforts de compression (F1), des stries (7) formant un angle (*α*1) inférieur à 90° par rapport à la fibre neutre (N) et se prolongeant du côté externe (EXT) de la fibre neutre (N) subissant des efforts de traction (F2), par des stries (8) formant un angle (α2) sensiblement égal à 90° par rapport à la même fibre neutre (N).

2. Tige fémorale (1) selon la revendication 1, **caractérisée en ce qu'**elle comporte sur sa face externe (EXT) rejoignant ses faces antéro postérieure (5, 6), des canelures rectilignes (10) s'étendant sur toute la longueur de la tige (1).

3. Tige fémorale selon l'une des revendications 1 ou 2, **caractérisée en ce que** sa face interne (INT) comporte sur sa partie métaphysaire (3) des stries transversales (11) sensiblement parallèles entre elles.

4. Tige fémorale selon l'une des revendications 1 à 3, **caractérisée en ce que** des canelures rectilignes (12) s'étendent sur les faces antéro postérieure (5, 6) dans la zone diaphysaire (2) au-delà des stries transversales (7, 8) de la zone métaphysaire (3).

5. Tige fémorale selon l'une des revendications 1 à 4, **caractérisée en ce qu'**il s'agit d'une tige droite.

6. Tige fémorale selon l'une des revendications 1 à 4, **caractérisée en ce qu'**il s'agit d'une tige anatomique.

## Claims

1. Femoral stem (1) for a total hip prosthesis, composed of a distal diaphysis part (2) that has an elongate shape and is designed to be engaged in the medullary cavity of a femur, with or without cement, and of a metaphysis part (3) whose free end (4) is provided with a head that is able to articulate in a cup, micro-reliefs being formed on at least one of the faces of the stem (1) in order to promote the regrowth of bone, **characterized in that** its antero-posterior faces (5, 6) have on their metaphysis part (3), on the internal side (INT) of the neutral axis (N), corresponding to the longitudinal axis (XY) of the stem (1), subject to compression forces (F1), striations (7) that form an angle (α1) of less than 90° relative to the neutral axis (N) and that are continued on the external side (EXT) of the neutral axis (N), subject to traction forces (F2), by striations (8) that form an angle (α2) substantially equal to 90° relative to the same neutral axis (N).

2. Femoral stem (1) according to Claim 1, **characterized in that**, on its external face (EXT) joining its antero-posterior faces (5, 6), it has rectilinear furrows (10) extending along the entire length of the stem (1).

3. Femoral stem according to one of Claims 1 and 2, **characterized in that** its internal face (INT) has, on its metaphysis part (3), transverse striations (11) substantially parallel to one another.

4. Femoral stem according to one of Claims 1 to 3, **characterized in that** rectilinear furrows (12) extend on the antero-posterior faces (5, 6) in the diaphysis zone (2) beyond the transverse striations (7, 8) of the metaphysis zone (3).

5. Femoral stem according to one of Claims 1 to 4, **characterized in that** it is a straight stem.

6. Femoral stem according to one of Claims 1 to 4, **characterized in that** it is an anatomical stem.

## Patentansprüche

1. Femurschaftprothese (1) für eine Totalprothese der Hüfte, bestehend aus einem distalen diaphysären Teil (2) mit länglicher Form, der dazu bestimmt ist, in den medulären Hohlraum eines Femurs eingesetzt zu werden, mit oder ohne Zement, und aus einem metaphysären Teil (3), dessen freier Endabschnitt (4) mit einem Kopf versehen ist, der zur gelenkigen Bewegung in einem Becher geeignet ist, wobei Mikroreliefs in zumindest einer der Flächen des Schafts (1) vorgesehen sind, um das Nachwachsen von Knochen zu begünstigen, **dadurch gekennzeichnet, daß** ihre vorderen und hinteren Flächen (5, 6) auf ihrem metaphysären Teil (3) auf der Innenseite (INT) der neutralen Faser (N), entsprechend der Längsachse (XY) des Schafts (1), kompressive Belastungen (F1) tragend, Riffeln (7) aufweist, die einen Winkel (α1) kleiner als 90° in Bezug auf die neutrale Faser (N) bilden und sich zur Außenseite (EXT) der neutralen Faser (N) fortsetzen, Zugbelastungen (F2) tragend, durch Riffeln (8), die einen Winkel (α2) von im wesentlichen gleich 90° in Bezug auf dieselbe neutrale Faser (N) bilden.

2. Femurschaftprothese (1) nach Anspruch 1, **dadurch gekennzeichnet, daß** sie auf ihrer Außenfläche (EXT), die sich an ihre vorderen und hinteren Flächen (5, 6) anschließt, geradlinige Rillen (10) aufweist, die sich über die gesamte Länge des Schafts (1) erstrecken.

3. Femurschaftprothese nach einem der Ansprüche 1 oder 2, **dadurch gekennzeichnet, daß** ihre Innenfläche (INT) auf ihrem metaphysären Teil (3) querverlaufende Riffeln (11) aufweist, die im wesentlichen untereinander parallel sind.

4. Femurschaftprothese nach einem der Ansprüche 1 bis 3, **dadurch gekennzeichnet, daß** sich geradlinige Rillen (12) auf den vorderen und hinteren Flächen (5, 6) in der diaphysären Zone (2) jenseits der quer verlaufenden Riffeln (7, 8) der metaphysären Zone (3) erstrecken.

5. Femurschaftprothese nach einem der Ansprüche 1 bis 4, **dadurch gekennzeichnet, daß** es sich um einen geraden Schaft handelt.

6. Femurschaftprothese nach einem der Ansprüche 1 bis 4, **dadurch gekennzeichnet, daß** es sich um einen anatomischen Schaft handelt.
